# EUROPEAN PATENT APPLICATION

(11) **EP 2 944 202 A1**
(43) Date of publication of application: **18.11.2015**
(21) Application number: 14168301.1
(22) Date of filing: 14.05.2014
(51) Int. Cl.: A23L 1/30, A23L 1/302, A23L 1/025, A61K 31/055, A61K 31/375, A61K 31/05

(54) **A method for manufacturing a mixture of lypo-spheric vitamin C and a mixture of lypo-spheric vitamin C**

(71) Applicant: Lypo C AB, 186 21 Vallentuna (SE)
(72) Inventor: Pollak, Asser, 169 53 Vallentuna (SE); Wallman, Johan, 186 43 Vallentuna (SE)

(57) **Abstract**

A method for manufacturing a mixture of lypo-spheric vitamin C. The method comprises the steps of:
- stirring vitamin C and resveratrol in water to a first mixture,
- stirring lecithin in water to a second mixture,
- stirring the first and the second mixture to a third mixture, and
- processing the third mixture by means of ultrasound until a smooth composition is obtained.

## Description

### FIELD OF THE INVENTION

The present invention relates to a method for manufacturing a mixture of lypo-spheric vitamin C. The method comprises stirring vitamin C and resveratrol in water to a first mixture, stirring lecithin in water to a second mixture, and stirring the first and the second mixture to a third mixture.

The present invention also relates to a mixture of lypo-spheric vitamin C manufactured according to the method.

### PRIOR ART

Mixtures of lypo-spheric vitamin C comprising vitamin C, resveratrol and lecithin in water are manufactured according to the state of the art by mixing and stirring vitamin C, resveratrol and lecithin in water. The mixture is intended to be consumed as a food supplement. A problem with the mixtures according to the state of the art is that vitamin C, resveratrol and lecithin can not be absorbed by the body in an optimal manner by the persons consuming the mixture.

### SUMMARY OF THE INVENTION

The object of the present invention is to provide a method for manufacturing a mixture of lypo-spheric vitamin C, where the absorption of vitamin C in the mixture by the consumer is improved compared with prior art mixtures.

The object is obtained with a method for manufacturing a mixture of lypo-spheric vitamin C according to claim 1. The method comprises the steps of:
- stirring vitamin C and resveratrol in water to a first mixture,
- stirring lecithin in water to a second mixture,
- stirring the first and the second mixture to a third mixture, and
- processing the third mixture by means of ultrasound until a smooth composition is obtained.

By processing the third mixture with ultrasound the substances of vitamin C, resveratrol and lecithin in the mixture is finely dispersed in the mixture, which results in improved absorption of the substances in the body of a person consuming the mixture. Accordingly, the ultrasound processing improves the function of the third mixture as a food supplement for avoiding deficiency of vitamin C.

According to an embodiment of the invention, the ultrasound processing of the third mixture comprises two of more steps with gradually increasing frequency. By means of the processing with gradually increasing frequency an improved dispersion of the components is obtained, which results in that the components can be better absorbed by the body of a person consuming the mixture.

According to an embodiment of the invention, the method comprises processing the third mixture at a frequency between 20-100 kHz.

According to an embodiment of the invention, the processing of the third mixture comprises a step comprising processing by means of ultrasound at a frequency between 30-40 kHz, preferably between 27-33 kHz.

According to an embodiment of the invention, the processing of the third mixture comprises a step comprising processing by means of ultrasound at a frequency between 40-50 kHz, preferably between 43-47 kHz.

According to an embodiment of the invention, the processing of the third mixture comprises a step comprising processing by means of ultrasound at a frequency between 50-60 kHz, preferably between 53-57 kHz.

According to an embodiment of the invention, the processing of the third mixture comprises a step comprising processing by means of ultrasound at a frequency between 60-70 kHz, preferably between 63-67 kHz.

According to an embodiment of the invention, the third mixture is processed in above indicated steps of the processing by means of ultrasound during a time period of between 10 and 30 minutes.

By means of the gradually processing the third mixture with gradually increasing frequencies according to one or more of the above indicated embodiments, agglomerates of the components vitamin C, reservatrol and lecithin are dispersed from larger agglomerates to finer agglomerates until the components essentially are dispersed separated in its molecules.

According to an embodiment of the invention, the components vitamin C, reservatrol and lecithin are added in form of powders.

According to an embodiment of the invention, the method comprises the formation of the first and the second mixture at a temperature above room temperature, preferably a temperature between 40 and 80 °C, more preferably between 55 and 65 °C.

According to an embodiment of the invention, the method comprises resting the third mixture during a time period between 12 and 48 hours, preferably between 18 and 28 hours.

According to an embodiment of the invention, the method comprises after the processing by means of ultrasound the steps of:
- stirring the third mixture, and
- bottling of the third mixture.

Above objects is also obtained with a mixture of lypo-spheric vitamin C manufactured by means of the method according to any of claim 1-9.

### BRIEF DESCRIPTION OF THE DRAWINGS

The invention will now be explained more closely by the description of different embodiments of the invention and with reference to the appended figure.

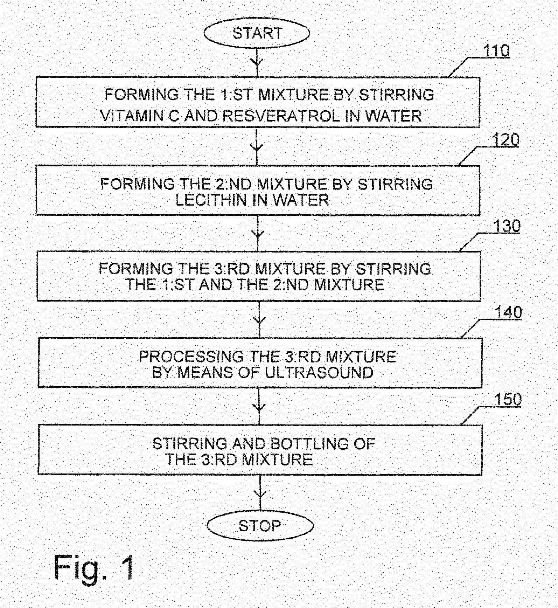

- Fig. 1: shows a flowchart over a method for manufacturing a mixture of lypo-spheric vitamin C.

### DETAILED DESCRIPTION OF PREFERRED EMBODIMENTS OF THE INVENTION

The method for manufacturing a mixture of lypo-spheric vitamin C will be explained with reference to the flow chart in fig. 1.

The method is initiated by means of that a first and a second mixture are formed. In a step 110 the first mixture is formed by adding vitamin C and resveratrol in form of powders to water at a temperature between 40 and 80 °C, preferably about 60 °C, and the vitamin C and the reservatrol are mixed in the water by stirring. In a step 120 the second mixture is formed by adding lecithin in form of powder to water at a temperature between 40 and 80 °C, preferably about 60 °C, and the lecithin is mixed in the water by stirring.

In a step 130, the first and the second mixture are brought together to a third mixture that is stirred. Thereafter, the third mixture is rested for about 24 hours.

In a step 140, the third mixture is processed by means of ultrasound using an ultra sonic cleaner until a smooth composition is obtained. The processing is initiated at a frequency of 35 kHz that thereafter gradually is stepped up to 45 kHz, 55 kHz and finally 70 kHz. By means of the gradually processing of the third mixture with gradually increasing frequency, the vitamin C, reservatrol and lecithin are finely dispersed in a manner that enable the components to be absorbed to a greater extent in the body of a person consuming the third mixture.

In a step 150, the third mixture is stirred and bottled. The third mixture is thereby ready to be consumed by the user.

The present invention is not limited to the disclosed embodiments but can be modified and varied within the framework of the subsequent claims.

## Claims

1. A method for manufacturing a mixture of lypo-spheric vitamin C, wherein the method comprises the steps of:
- stirring vitamin C and resveratrol in water to a first mixture,
- stirring lecithin in water to a second mixture,
- stirring the first and the second mixture to a third mixture, and
- processing the third mixture by means of ultrasound until a smooth composition is obtained.

2. The method according to claim 1, wherein the processing of the third mixture comprises processing at a frequency between 20-100 kHz.

3. The method according to claim 1 and 2, wherein the processing of the third mixture comprises:
- a step comprising processing by means of ultrasound at a frequency between 30-40 kHz, preferably between 27-33 kHz.

4. The method according to any of the previous claims, wherein the processing of the third mixture comprises:
- a step comprising processing by means of ultrasound at a frequency between 40-50 kHz, preferably between 43-47 kHz.

5. The method according to any of the previous claims, wherein the processing of the third mixture comprises:
- a step comprising processing by means of ultrasound at a frequency between 50-60 kHz, preferably between 53-57 kHz.

6. The method according to any of the previous claims, wherein the processing of the third mixture comprises:
- a step comprising processing by means of ultrasound at a frequency between 60-70 kHz, preferably between 63-67 kHz.

7. The method according to any of the previous claims, wherein the formation of the first and the second mixture take place at a temperature above room temperature, preferably a temperature between 40 and 80 °C, more preferable between 55 and 65 °C.

8. The method according to any of the previous claims, wherein the method comprises:
- resting the third mixture during a time period between 12 and 48 hours, preferably between 18 and 28 hours.

9. The method according to any of the previous claims, wherein the method comprises the steps:
- stirring the third mixture, and
- bottling the third mixture.

10. A mixture of lypo-spheric vitamin C manufactured by means of the method according to any of claim 1-9.
